(19) Europäisches Patentamt
European Patent Office
Office européen des brevets

(11) Publication number: **0 422 803 A2**

# (12) EUROPEAN PATENT APPLICATION

(21) Application number: **90310633.4**

(51) Int. Cl.[5]: **A61K 7/16**

(22) Date of filing: **28.09.90**

The title of the invention has been amended (Guidelines for Examination in the EPO, A-III, 7.3).

(30) Priority: **11.10.89 GB 8922920**
**11.10.89 GB 8922921**

(43) Date of publication of application:
**17.04.91 Bulletin 91/16**

(84) Designated Contracting States:
**AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Alexander, Stephen Edward,**
**SmithKline Beecham Con.**
**Brands, St. George's Avenue**
**Weybridge, Surrey KT13 0DE(GB)**
Inventor: **Doel, Geoffrey Royston, SmithKline Beecham Con.**
**Brands, St. George's Avenue**
**Weybridge, Surrey KT13 0DE(GB)**
Inventor: **Edwards, Peter John, SmithKline Beecham Con.**
**Brands, St. George's Avenue**
**Weybridge, Surrey KT13 0DE(GB)**

(74) Representative: **Lockwood, Barbara Ann et al**
**SmithKline Beecham, Corporate Patents,**
**Great Burgh, Yew Tree Bottom Road**
**Epsom, Surrey KT18 5XQ(GB)**

## (54) Dentifrice comprising cationic antibacterial agent.

(57) The problem of formulating a dentifrice for use in treating periodontal disease, calculus and/or caries and comprising a cationic antibacterial agent is overcome by combining a nonionic thickening agent, a nonionic, cationic or amphoteric surfactant and an abrasive which is either a sparingly soluble salt, in combination with an agent to suppress anion formation, or an essentially insoluble compound such as silica, preferably with an anion content of less than 1%.

EP 0 422 803 A2

## COMPOSITION

The present invention relates to a dentifrice composition comprising an antibacterial agent, in particular a cationic antibacterial agent, which compositions are useful in the prophylaxis and/or treatment of periodontal disease, calculus and caries.

The use of antibacterial agents, including cationic antibacterial agents, in oral hygiene compositions has been widely advocated as a means of reducing the bacterial plaque population and this may be beneficial in the treatment of peridontal disease, calculus, and/or caries.

Whilst mouthwashes comprising cationic antibacterial agents are available, these suffer the disadvantage that the cationic antibacterial agents tend to leave a brown stain, due to interaction of the agent with plaque. Such a drawback may, in principle, be minimised by using the antibacterial agent in a dentifrice, so that the abrasive included therein may remove the plaque. In practise however there are found to be severe problems in providing a satisfactory formulation because of the intrinsic incompatability of cationic antibacterial agents with many of the other conventional elements of a dentifrice formulation, which incompatibility drastically reduces the biological activity of the cationic agent. In addition, the cationic antibacterial agents are recognised to have a bitter taste which needs to be masked, to provide a product which is acceptable to the consumer.

EP-A-0 364 245 (to Beecham Group plc, and published after the priority date of the present application) discloses dentifrices comprising a bis-biguanide antibacterial such as chlorhexidine in combination with a nonionic surfactant, a nonionic thickening agent and an abrasive selected for compatibility with the antibacterial agent, for instance a silica with a low anion content. In addition EP-A-0 368 130 (to Procter ϵ Gamble Co, and published after the priority date of the present application) discloses generically dentifrices comprising a cationic antibacterial agent in combination with a nonionic surfactant, a nonionic thickening agent, a nonionic humectant and a silica abrasive having good compatibility with cationic antibacterial agents. The specific examples provided therein are of a chorhexidine-containing dentifrice of which the compatible silica is a special experimental grade provided by J.M. Huber Corporation and characterised by inter alia a low sulphate ion content (less than 0.25%), a BET a surface area of about 10 to 300 $m^2g^{-1}$ and the presence of from 10 to 300 parts per million of alkaline earth metal ions. The latter are introduced during the final stage of preparation of the silica, to produce a special surface-coated silica.

EP-A-0 315 503 (to Rhone-Poulenc Chimie) discloses the suitability of certain grades of silicas for use in chlorhexidine-containing dentifrices, which silicas are characterised by inter alia , a low anion content (less than 1%). No teaching is however provided concerning the selection of suitable other compatible components, such as the surfactant or thickening agent. US 4 273 759 (to Colgate-Palmolive Co.) discloses dentifrices comprising a cationic antibacterial agent such as chlorhexidine or cetyl pyridinium chloride in combination with an anti-staining agent to counter the stain associated with the use of the cationic antibacterial agent. In addition, Examples 18 to 20 thereof disclose formulations comprising Pluronic F-108 as the surfactant and hydroxypropyl methylcellulose as the thickening agent.

Several methods of overcoming the unpleasant bitter flavour associated with the use of a cationic antibacterial agent have been disclosed, including the use, in an alumina-based dentifrice, of a predominantly peppermint flavour, moderated by the addition of an aniseed flavour (GB 2 035 084, to Unilever).

It has now been discovered that the problem of formulating a cationic antibacterial agent to overcome or at least mitigate the problem of incompatibility may be solved by the use of a particular formulation of dentifrice.

Accordingly, the present invention provides a dentifrice comprising:

a bacteriostatically effective amount of a cationic antibacterial agent;

a nonionic thickening agent;

a nonionic, cationic or amphoteric surfactant, or a mixture thereof; and

an abrasive which comprises either at least one sparingly soluble salt, in combination with an agent to suppress anion formation, or at least one essentially insoluble compound, or a mixture thereof;

and excluding a dentifrice in which the surfactant is essentially a nonionic surfactant and the cationic antibacterial agent is a bis-biguanide and a dentifrice in which the surfactant is essentially a nonionic surfactant and the abrasive consists essentially of a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$, calcium pyrophosphate, trimagnesium phosphate, alumina, hydrated alumina, an aluminium silicate or a mixture thereof.

Suitable cationic antibacterial agents for use in dentifrices of the invention include, for example:

(i) quaternary ammonium compounds, for instance those in which one or two of the substituents on the quaternary nitrogen has from 8 to 20, preferably from 10 to 18 carbon atoms and is preferably an alkyl group, which may optionally be interrupted by an amide, ester, oxygen, sulphur, or heterocyclic ring, whilst the remaining substituents have a lower number of carbon atoms, for instance from 1 to 7, and are preferably alkyl, for instance methyl or ethyl, or benzyl, examples of such compounds including benzalkonium chloride, dodecyl trimethyl ammonium chloride, benzyl dimethyl stearyl ammonium chloride, and cetyl trimethyl ammonium bromide, benzethonium chloride (diisobutyl phenoxyethoxyethyl dimethyl benzyl ammonium chloride), and methyl benzethonium chloride;

(ii) pyridinium and isoquinolinium compounds, including hexadecylpyridinium chloride, cetyl pyridinium chloride, and alkyl isoquinolinium bromide;

(iii) pyrimidine derivatives such as hexetidine (5-amino-1,3-bis(2-ethylhexyl)-5-methylhexahydropyrimidine);

(iv) amidine derivatives such as hexamidine isethionate (4,4′-diamidino-α-,ω-diphenoxyhexane isethionate);

(v) bispyridine derivatives such as octenidine (N,N′[1,10-decanediyldi-1(4H)-pyridinyl-4-ylidene]-bis (1-octanamine dihydrochloride); and

(vi) guanides, for example, mono-biguanides such as p-chlorobenzyl-biguanide and N′-(4-chlorobenzyl)-N″-(2,4-dichlorobenzyl)biguanide, poly(biguanides) such as polyhexamethylene biguanide hydrochloride, and bis-biguanides of the general formula (1):

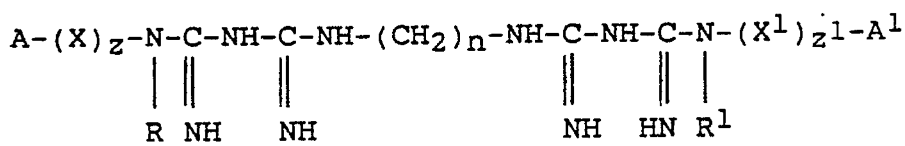

(I)

in which

A and $A^1$ each represent (i) a phenyl group optionally substituted by $(C_{1-4})$alkyl, $(C_{1-4})$alkoxy, nitro, or halogen, (ii) a $(C_{1-12})$alkyl group, or (iii) a $(C_{4-12})$-alicyclic group;

X and $X^1$ each represent $(C_{1-3})$alkylene;

R and $R^1$ each represent hydrogen, $(C_{1-12})$alkyl, or aryl$(C_{1-6})$alkyl;

Z and $Z^1$ are each 0 or 1;

n is an integar from 2 to 12;

and the polymethylene chain $(CH_2)_n$ may optionally be interrupted by oxygen or sulphur or an aromatic (for instance phenyl or naphthyl) nucleus; and

orally acceptable acid addition salts thereof; examples of such bis-biguanides including chlorhexidine and alexidine.

Suitable acid addition salts of the bis-biguanides of formula (I) include the diacetate, the dihydrochloride and the digluconate.

Suitable acid addition salts of chlorhexidine are those which have a water solubility at 20°C of at least 0.005% w/v and include the digluconate, diformate,diacetate, dipropionate, dihydrochloride, dihydroiodide, dilactate, dinitrate, sulphate, and tartrate salts. Preferably the salt is the dihydrochloride, diacetate or digluconate salt.

Suitable acid addition salts of alexidine include the dihydrofluoride and the dihydrochloride salt.

Suitably, the cationic antibacterial agent is selected from benzethonium chloride, octenidine, hexetidine, hexamidine, cetyl pyridinium chloride, chlorhexidine or alexidine.

Advantageously, the cationic antibacterial agent is present in the range 0.005 to 10%, preferably 0.005 to 5%, more preferably 0.005 to 2.5% by weight of the dentifrice.

Suitable nonionic thickening agents include, for example, $(C_{1-6})$alkylcellulose ethers, for instance methylcellulose, hydroxy$(C_{1-6})$alkylcellulose ethers, for instance hydroxypropylcellulose, $(C_{2-6})$alkylene oxide modified $(C_{1-6})$alkylcellulose ethers, for instance hydroxypropyl methylcellulose, and mixtures thereof.

Advantageously the nonionic thickening agent is present in the range 0.01 to 30%, preferably 0.1 to 15%, more preferably 1 to 5%, by weight of the dentifrice.

Suitable nonionic surfactants include, for example polyethoxylated sorbitol esters in particular

polyethoxylated sorbitol monoesters, for instance, PEG(40) sorbitan diisostearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc.

Advantageously, the surfactant is present in the range 0.005 to 20%, preferably 0.1 to 10%, more preferably 0.1 to 5% by weight of the dentifrice.

Suitable sparingly soluble salts that may be used as an abrasive include calcium carbonate, calcium phosphates, magnesium carbonate, insoluble sodium metaphosphate, and suitable mixtures thereof. The agent to suppress anion formation typically comprises a water soluble salt containing a cation which may be same as the cation of the abrasive and which forms an essentially insoluble or sparingly soluble salt with the anion of the abrasive.

Preferably the sparingly soluble salt used as an abrasive is calcium carbonate, advantageously used in combination with dicalcium phosphate, which also usefully buffers the pH of the formulation. Suitable types of calcium carbonate include both natural and synthetic chalks.

The agent to suppress anion formation may be an alkaline earth metal salt, for instance calcium chloride. The agent is preferably present in from 0.0001 to 1%, more preferably 0.005 to 0.1% by weight of the dentifrice.

The term 'essentially insoluble compound' as used herein refers to a compound which is intrinsically insoluble in aqueous solution and includes those compounds which are listed as being 'insoluble' in cold water in the 'Handbook of Chemistry and Physics', 48th Edition, Chemical Rubber Company, Section B, Physical Constants of Inorganic Compounds. Furthermore, such compounds when used as an abrasive shall contain little if any contaminating anionic impurities. Preferably the insoluble abrasive compound contains less than 1%, more preferably less than 0.5%, of anionic impurities, based on the weight of the abrasive.

Suitable essentially insoluble compounds for use as abrasives include, for example, silica, zinc orthophosphate, plastics particles, alumina, hydrated alumina, and calcium pyrophosphate or mixtures thereof; of which silica is preferred.

The preferred silica abrasive may be a natural amorphous silica, for instance diatomaceous earth; or a synthetic amorphous silica, for instance a precipitated silica, or a silica gel, such as a silica xerogel; or mixtures thereof.

The preferred grades of synthetic amorphous silicas are those for which the manufacturing process is carefully controlled so that the level of anion impurities, particularly sulphate and silicate from sodium sulphate and sodium silicate, respectively, is kept to a minimum. Alternatively, or in addition, the level of anion impurities may be reduced to the required level by careful washing of the silica with, for instance, deionised or distilled water.

Suitable silicas include those described in EP-A-0 315 503 (to Rhone-Poulenc) which are characterised as having:

(i) less than 5 X $10^{-3}$, preferably less than 1 X $10^{-3}$, more preferably less than 0.2 x $10^{-3}$ moles of anions per 100g of silica, and in particular less than 0.5%, preferably less than 0.1%, more preferably less than 0.02% by weight of silica of sulphate anions;

(ii) a Hammett acidity function Ho of at least 3.3, which may be determined according to the method of Walling, J. Amer. Chem. Soc. , 1950, 72 , 1164;

(iii) a surface in which the number of hydroxyl groups, expressed as OH/$nm^2$ is less than 15, and more particularly less than 12, which may be determined according to the method described in EP-A-0 315 503; and

(iv) a point zero charge of at least 3 and preferably between 4 and 6, which point zero charge may be determined according to the method described in EP-A-0 315 503.

Such silicas may be prepared as precipitates or gels from silicate and acid by conventional means, the crude silica product being collected and then washed with water, preferably deionised water, until the conductivity of the washings is less than 200 microsiemens $cm^{-1}$, preferably less than 100 microsiemens $cm^{-1}$, and then dried and, if necessary, ground, to give the desired sizes of particles. Alternatively, an initial washing of the crude product with water, until the washings have a conductivity of less than 2000

microsiemens $cm^{-1}$, may be followed by washing with an acid or an aqueous acid, for instance a mineral acid such as nitric acid, or an organic acid such as acetic acid or citric acid, until the silica has a pH of less than 8, preferably between 6 and 7.5.

Suitable silica xerogels are described in US 3 538 230.

Preferred precipitated silicas are those marketed under the trade name 'SIDENT' by Degussa, for instance, SIDENT 9 silica. Preferred silica xerogels are those marketed under the trade name 'SYLOBLANC' by W.R. Grace Corporation, Davison Chemical Division.

Suitable grades of precipitated silica have BET surface areas in the range 20 to 300, preferably 20-100 $m^2/g$ and median agglomerate sizes in the range 2 to 50, preferably 5 to 30$\mu$.

Suitable forms of diatomaceous earth include those marketed under the trade name 'Celite' by Johns-Manville Products Corporation, for instance 'Celite Superfine Superfloss'.

Diatomaceous earth may be employed on its own or it may be used in combination with a synthetic amorphous silica, in particular a precipitated silica (as hereinbefore defined), which also usefully buffers the pH of the formulation and counters the otherwise off-white colour and roughness conferred upon a formulation by the use of diatomaceous earth alone. Suitably, the ratio of diatomaceous earth to synthetic amorphous silica is from 5:1 to 1:5, preferably about 1:1.

The abrasive is advantageously present in the range 1-80%, preferably 5-70%, more preferably 5-60% by weight of the dentifrice.

It will be appreciated that each of the nonionic thickening agent, the nonionic, cationic or amphoteric surfactant and abrasive should be, at the level employed in the dentifrice, compatible with the cationic antibacterial agent, that is, each will not substantially reduce the availability of the cationic antibacterial agent, for instance, by more than 30%, preferably more than 20%. This may be confirmed by, for instance, determining the biological activity of the formulation, by conventional microbiological assay using, for instance, m . luteus as the assay organism in a standard agar diffusion method, in the presence and absence of each of the aforementioned thickening agent, surfactant and abrasive.

In another aspect of this invention it has been found that in certain embodiments, a dentifrice according to the invention may further comprise an ionic fluorine-containing compound, which may include ionic fluorides, such as alkali metal fluorides, and ionic monofluorophosphates, such as alkali metal monofluorophosphates, and which may be incorporated into the formulation, to provide between 100 and 3000ppm, preferably 500 to 2000ppm of fluoride. Preferably the ionic fluoride or monofluorophosphate is an alkali metal fluoride or mcnofluorophosphate, for instance sodium fluoride or sodium monofluorophosphate, respectively.

It will be appreciated that the ionic fluorine-containin compound should be compatible with the cationic antibacterial agent; that is, the agent will not substantially reduce the availability of the cationic antibacterial agent, for instance by more than 30%, preferably more than 20%. This may be confirmed by, for instance, determining the biological activity of the formulation, by conventional microbiological assay using, for instance, m . luteus as the assay organism in a standard agar diffusion method, in the presence and absence of the ionic fluorine-containing compound

Thus, for instance, sodium fluoride is found to be compatible with chlorhexidine, alexidine, cetyl pyridinium chloride, benzenthonium chloride and benzalkonium chloride and sodium monofluorophosphate is found to be compatable with hexamidine.

It will further be appreciated that if an ionic fluorine-containing compound is incorporated in a dentifrice of the invention, the abrasive should be chosen so that it is compatible with the ionic fluorine-containing compound. Thus, for instance, sodium fluoride is well known in the art to be incompatible with abrasives with comprise excess calcium ions as these cause loss of fluoride as insoluble calcium fluoride. Accordingly an abrasive which is insoluble, for instance, a silica, alumina, zinc orthophosphate or plastics particles, is preferred.

Dentifrices according to the invention may also contain a humectant, such as glycerine, sorbitol, propylene glycol or polyethylene glycol, or mixtures thereof; which humectant may be present in the range from 5 to 70%, preferably 5 to 30%, more preferably 10 to 30% by weight of the dentifrice. Suitably, when the nonionic thickening agent is hydroxypropyl methylcellulose, the humectant is present in up to 30% by weight of the dentifrice.

Dentifrices according to the invention may also contain other agents conventionally used in dentifrice formulations, for instance flavouring agents; colouring agents; whitening agents; preservatives and sweetening agents. It will be appreciated that such agents (at the level employed) should be compatible with the cationic antibacterial agent; that is, the agent will not substantially reduce the availability of the cationic antibacterial agent, for instance by more than 30%, preferably more than 20%. This may be confirmed as hereinbefore discussed. In general, such agents will be a minor amount or proportion of the formulation,

usually present in from 0.001 to 5% by weight of the composition.

Flavour is an important aspect of the consumer acceptability of a dentifrice. This is particularly so in the case of a dentifrice comprising a cationic antibacterial agent, because of the bitter after-taste of the cationic antibacterial agent. Surprisingly it has now been found that this can be effectively masked by an aniseed flavour.

Accordingly, in a further aspect, the present invention provides a dentifrice as hereinbefore defined which further comprises a flavouring agent having an aniseed flavour.

Flavouring agents having an aniseed flavour include anethole, which may be present in such quantity as to mask the bitter after-taste of the cationic antibacterial agent.

Preferably the flavour may be modified by the additional incorporation of one or more flavouring agents having a mint flavour, to balance the aniseed flavour, to give a flavour which has more general consumer acceptability but in which the aniseed flavour is still dominant.

Suitable flavouring agents having a mint flavour include peppermint, spearmint, menthol and carvone.

Preferably more than one mint flavouring agent is used, of which menthol may be the major component, accounting for between 20 and 60%, preferably between 25 and 55% by weight of the flavouring agents having a mint flavour.

Advantageously the flavour of the dentifrice may be further modified by the incorporation of flavouring agents having spicey flavours such as coriander, eugenol and eucalyptol, the aniseed note still being dominant.

Accordingly, in a still further aspect, the invention provides a dentifrice as hereinbefore defined comprising aniseed and mint flavours and, optionally, spicey flavours and having a predominantly aniseed flavour.

Preferably, in such a dentifrice comprising aniseed and mint flavours, flavouring agents having an aniseed flavour comprise from 10 to 30%, more preferably 15 to 25% of the combined weights of the flavouring agents, whilst flavouring agents having a mint flavour comprise from 40 to 80, preferably 40 to 70% of the combined weight of the flavouring agents. Preferably the combined flavouring agents comprise up to 5%, more preferably up to 2% by weight of the dentifrice.

Suitable sweetening agents include saccharin, cyclamate and acesulfame, and acceptable water soluble salts thereof, such as an alkali metal salt, and may be present in from 0.01 to 0.5%, preferably 0.05 to 0.5% by weight of the dentifrice. An auxiliary sweetener such as a thaumatin may also be included, at a level of from 0.001 to 0.1, preferably 0.005 to 0.05% by weight of the dentifrice. A suitable blend of thaumatins is marketed under the trade name 'TALIN' by Tate and Lyle plc.

Water, preferably deionised or distilled water, will also be present, in the range from 10 to 80%, preferably 20 to 70% by weight of the dentifrice.

Dentifrices according to the invention may also contain an antistain agent. Suitable antistain agents include, for example, carboxylic acids such as those disclosed in US 4 256 731, amino carboxylate compounds such as those disclosed in US 4 080 441 and phosphonoacetic acid, as disclosed in US 4 118 474. The antistain agent may be incorporated into the dentifrice formulation or may be provided as a separate composition, for use after the dentifrice.

Dentifrices according to the invention may also usefully contain a zinc ion source, for instance an organic or an inorganic salt thereof, such as the acetate, benzoate, citrate, lactate, tartrate glycerophosphate, nitrate, chloride and sulphate. Preferably, the zinc ion source will provide from 0.1 to 1.5%, more preferably 0.2 to 0.5%, of zinc ions.

The dentifrices according to the invention may have a pH of less than 10 and preferably within the range pH 4 to 10, most preferably pH 5 to 8.

In a preferred aspect, the present invention provides a dentifrice comprising a bacteriostatically effective amount of cetyl pyridinium chloride, a nonionic thickening agent which is preferably hydroxypropyl methylcellulose, a nonionic surfactant which is preferably a polycondensate of ethylene oxide and propylene oxide, and an abrasive which is an essentially insoluble compound, and preferably a synthetic amorphous silica as hereinbefore defined.

The dentifrices according to the invention may be prepared in a conventional manner by mixing the ingredients thereof in the required proportions and in any order which is convenient and, thereafter and if necessary, adjusting the pH. For instance, the nonionic thickening agent and the humectant and part of the water are vigourously agitated together, with heat, if necessary, to give a hydrated gel. Abrasive is then dispersed in this hydrated gel, using a heavy-duty mixing machine, with active agents, such as the cationic antibacterial agent, a fluoride salt (if present), then added, followed by surfactant and flavouring agents in the final stage; with final mixing carried out under vacuum.

The invention also provides a method for the prophylaxis or treatment of periodontal disease, calculus

and/or caries, which method comprises applying a composition according to the invention to the oral cavity.

The following examples illustrate the invention.

| Example 1 | |
|---|---|
| | % |
| Glycerine | 22.0 |
| Hydroxypropyl methylcellulose | 3.6 |
| Chalk | 32.0 |
| Dicalcium phosphate | 3.0 |
| Calcium chloride | 0.01 |
| Saccharin | 0.10 |
| Thaumatin | 0.02 |
| Flavour | 1.00 |
| Benzethonium chloride | 1.00 |
| Poloxamer 338 | 2.00 |
| Water (deionised) | to 100.00 |

| Example 2 | |
|---|---|
| | % |
| Glycerine | 22.0 |
| Hydroxypropyl methylcellulose | 3.6 |
| Diatomaceous earth | 8.0 |
| Silica[a] | 8.0 |
| Saccharin | 0.10 |
| Thaumatin | 0.02 |
| Flavour | 1.00 |
| Sodium flouride | 0.22 |
| Benzethonium chloride | 1.00 |
| Poloxamer 338 | 2.00 |
| Water (deionised) | to 100.00 |

[a]Sident 9

| Example 3 | |
|---|---|
| | % |
| Glycerine | 22.0 |
| Hydroxypropyl methylcellulose | 3.6 |
| Silica[a] | 16.0 |
| Saccharin | 0.10 |
| Thaumatin | 0.02 |
| Flavour | 1.00 |
| Sodium flouride | 0.22 |
| Octenidine | 1.00 |
| Poloxamer 338 | 2.00 |
| Water (deionised) | to 100.00 |

[a]Sident 9

| Example 4 | % |
|---|---|
| Glycerine | 22.0 |
| Hydroxypropyl methylcellulose | 3.6 |
| Diatomaceous earth | 8.0 |
| Silica[a] | 8.0 |
| Saccharin | 0.10 |
| Thaumatin | 0.02 |
| Flavour | 1.00 |
| Sodium flouride | 0.22 |
| Hexetidine | 1.00 |
| Poloxamer 338 | 2.00 |
| Water (deionised) | to 100.00 |

[a]Sident 9

| Example 5 | % |
|---|---|
| Glycerine | 22.0 |
| Hydroxypropyl methylcellulose | 3.6 |
| Silica[a] | 16.0 |
| Saccharin | 0.10 |
| Thaumatin | 0.02 |
| Flavour | 1.00 |
| Sodium monofluorophosphate | 0.76 |
| Hexemidine | 1.00 |
| Poloxamer 338 | 2.00 |
| Water (deionised) | to 100.00 |

[a]Sident 9

| Example 6 | % |
|---|---|
| Glycerine | 22.0 |
| Hydroxypropyl methylcellulose | 3.6 |
| Silica[a] | 16.0 |
| Saccharin | 0.10 |
| Thaumatin | 0.02 |
| Flavour | 1.00 |
| Sodium fluoride | 0.22 |
| Cetyl pyridinium chloride | 1.00 |
| Poloxamer 338 | 2.00 |
| Water (deionised) | to 100.00 |

[a]Sident 9

| Example 7 | |
|---|---|
| | % |
| Glycerine | 18.0 |
| Hydroxypropyl methylcellulose | 3.2 |
| Alumina | 46.0 |
| Saccharin | 0.10 |
| Thaumatin | 0.02 |
| Flavour | 1.00 |
| Sodium monofluorophosphate | 1.00 |
| Hexamidine | 0.76 |
| CAE* | 2.00 |
| Water (deionised) | to 100.00 |

*D,L-2-pyrrolidone-5-carboxylic acid salt of ethyl N-cocoyl-L-arginate.

| Example 8 | |
|---|---|
| | % |
| Glycerine | 22.0 |
| Hydroxypropyl methylcellulose | 3.6 |
| Chalk | 32.0 |
| Dicalcium phosphate | 3.0 |
| Calcium chloride | 0.01 |
| Saccharin | 0.10 |
| Thaumatin | 0.02 |
| Flavour | 1.00 |
| Octenidine | 1.00 |
| Alkyl dimethyl betaine | 2.00 |
| Water (deionised) | to 100.00 |

| Example 9 | |
|---|---|
| | % |
| Glycerine | 22.0 |
| Hydroxypropyl methylcellulose | 3.6 |
| Diatomaceous earth | 8.0 |
| Silica[a] | 8.0 |
| Acesulpham K | 0.3 |
| Flavour | 1.00 |
| Sodium fluoride | 0.22 |
| Chlorhexidine gluconate | 1.00 |
| CAE | 2.00 |
| Water (deionised) | to 100.00 |

[a]Sident 9

| Example 10 | |
|---|---|
| | % |
| Glycerine | 22.0 |
| Hydroxypropyl methylcellulose | 3.6 |
| Silica[a] | 16.0 |
| Saccharin | 0.10 |
| Flavour | 1.00 |
| Sodium fluoride | 0.22 |
| Octenidine | 1.00 |
| Alkyl dimethyl betaine | 2.00 |
| Water (deionised) | to 100.00 |

[a]Sident 9

| Example 11 | |
|---|---|
| | % |
| Glycerine | 22.0 |
| Hydroxypropyl methylcellulose | 3.6 |
| Diatomaceous earth | 8.0 |
| Silica[a] | 8.0 |
| Saccharin | 0.10 |
| Flavour | 1.00 |
| Sodium fluoride | 0.22 |
| Benzethonium chloride | 1.00 |
| CAE | 2.00 |
| Water (deionised) | to 100.00 |

[a]Sident 9

## Claims

1. A dentifrice comprising:
a bacteriostatically effective amount of a cationic antibacterial agent;
a nonionic thickening agent;
a nonionic, cationic or amphoteric surfactant, or a mixture thereof; and
an abrasive which comprises either at least one sparingly soluble salt, in combination with an agent to suppress anion formation, or at least one essentially insoluble compound, or a mixture thereof;
and excluding a dentifrice in which the surfactant is essentially a nonionic surfactant and the cationic antibacterial agent is a bis-biguanide and a dentifrice in which the surfactant is essentially a nonionic surfactant and the abrasive consists essentially of a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$, calcium pyrophosphate, trimagnesium phosphate, alumina, hydrated alumina, an aluminium silicate or a mixture thereof.

2. A dentifrice as claimed in claim 1 in which the cationic antibacterial agent is selected from a quaternary ammonium compound, a pyridinium or a isoquinolinium compound, a pyrimidine derivative, an amidine derivative, a bispyridine derivative, a mono-biguanide, a poly(biguanide) or bis-biguanide of the general formula (I):

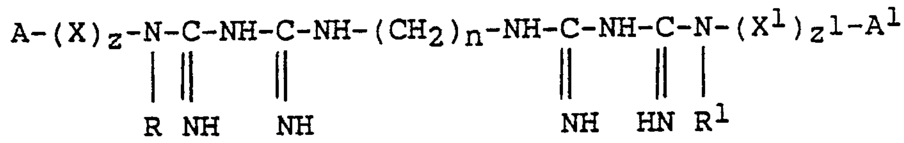

(I)

in which
A and $A^1$ each represent (i) a phenyl group optionally substituted by $(C_{1-4})$alkyl, $(C_{1-4})$alkoxy, nitro, or halogen, (ii) a $(C_{1-12})$alkyl group, or (iii) a $(C_{4-12})$-alicyclic group;
X and $X^1$ each represent $(C_{1-3})$alkylene;
R and $R^1$ each represent hydrogen, $(C_{1-12})$alkyl, or aryl$(C_{1-6})$alkyl;
Z and $Z^1$ are each 0 or 1;
n is an integar from 2 to 12;
and the polymethylene chain $(CH_2)_n$ may optionally be interrupted by oxygen or sulphur or an aromatic (for instance phenyl or naphthyl) nucleus; or an orally acceptable acid addition salt thereof.

3. A dentifrice as claimed in claim 1 or claim 2 in which the cationic antibacterial agent is selected from benzethonium chloride, octenidine, hexetidine, hexamidine, cetyl pyridinium chloride, chlorhexidine or alexidine.

4. A dentifrice as claimed in any one of claims 1 to 3 in which the nonionic thickening agent is selected from a $(C_{1-6})$alkylcellulose ether, a hydroxy$(C_{1-6})$alkylcellulose ether, a $(C_{2-6})$alkylene oxide modified $(C_{1-6})$alkylcellulose ether, or a mixture thereof.

5. A dentifrice as claimed in any one of claims 1 to 4 in which the nonionic surfactant is selected from a polyethoxylated sorbitol ester, a polycondensate of ethylene oxide and propylene oxide, a condensate of propylene glycol, a polyethoxylated hydrogenated castor oil or a sorbitan fatty ester; the amphoteric surfactant is selected from a long chain imidazoline derivative, a long chain alkyl betaine or a long chain alkyl amidoalkyl betaine; or the cationic surfactant is the D,L-2-pyrrolidone-5-carboxylic acid salt of ethyl-N-cocoyl-L-arginate.

6. A dentifrice as claimed in any one of claims 1 to 5 in which the abrasive is an essentially insoluble compound selected from silica, zinc orthophosphate, plastic particles, alumina, hydrated alumina, and calcium pyrophosphate.

7. A dentifrice as claimed in any one of claims 1 to 6 in which the essentially insoluble compound used as the abrasive compound contains less than 1% of anionic impurities, based on the weight of the abrasive.

8. A dentifrice as claimed in claim 7 in which the essentially insoluble compound contains less than 0.5% of anionic impurities, based on the weight of the abrasive.

9. A dentifrice as claimed in any one of claims 1 to 8 in which the abrasive is a natural amorphous silica or a synthetic amorphous silica, or a mixture thereof.

10. A dentifrice as claimed in claim 9 in which the silica comprises less the 0.5%, by weight of silica, of sulphate anions.

11. A dentifrice as claimed in any one of claims 1 to 5 in which the abrasive is a sparingly soluble salt selected from calcium carbonate, calcium phosphates, magnesium carbonate, insoluble sodium metaphosphate, or a mixture thereof.

12. A dentifrice as claimed in any one of claims 1 to 5 and 11 in which the agent to suppress anion formation comprises a water soluble salt containing a cation which is the same as the cation of the abrasive and which forms an essentially insoluble or sparingly soluble salt with the anion of the abrasive.

13. A dentifrice as claimed in any one of claims 1 to 12 which further comprises an ionic fluorine-containing compound.

14. A dentifrice as claimed in any one of the preceding claims which further comprises a flavouring agent having an aniseed flavour.

15. A dentifrice comprising a bacteriostatically effective amount of cetyl pyridinium chloride, a nonionic thickening agent, a nonionic surfactant and an abrasive which is a synthetic amorphous silica comprising less than 1% of anionic impurities, and excluding a dentifrice comprising a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$.

16. A process for preparing a dentifrice according to any one of the preceding claims which process

comprises mixing the ingredients thereof in the required proportions and in any order which is convenient and, thereafter and if necessary, adjusting the pH.

17. The use of a dentifrice according to any one of claims 1 to 15 in oral hygiene.

Claims for the following Contracting State: ES

1. A process for preparing a dentifrice comprising:
a bacteriostatically effective amount of a cationic antibacterial agent;
a nonionic thickening agent;
a nonionic, cationic or amphoteric surfactant, or a mixture thereof; and
an abrasive which comprises either at least one sparingly soluble salt, in combination with an agent to suppress anion formation, or at least one essentially insoluble compound, or a mixture thereof;
and excluding a dentifrice in which the surfactant is essentially a nonionic surfactant and the cationic antibacterial agent is a bis-biguanide and a dentifrice in which the surfactant is essentially a nonionic surfactant and the abrasive consists essentially of a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$, calcium pyrophosphate, trimagnesium phosphate, alumina, hydrated alumina, an aluminium silicate or a mixture thereof; which process comprises preparing of a dentifrice according to any one of the preceding claims which process comprises mixing the ingredients thereof in the required proportions and in any order which is convenient and, thereafter and if necessary, adjusting the pH.

2. A process as claimed in claim 1 in which the cationic antibacterial agent is selected from a quaternary ammonium compound, a pyridinium or a isoquinolinium compound, a pyrimidine derivative, an amidine derivative, a bispyridine derivative, a mono-biguanide, a poly(biguanide) or bis-biguanide of the general formula (I):

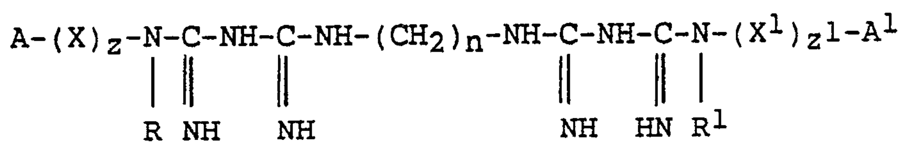

(I)

in which
A and $A^1$ each represent (i) a phenyl group optionally substituted by $(C_{1-4})$alkyl, $(C_{1-4})$alkoxy, nitro, or halogen, (ii) a $(C_{1-12})$alkyl group, or (iii) a $(C_{4-12})$-alicyclic group;
X and X1 each represent $(C_{1-3})$alkylene;
R and R1 each represent hydrogen, $(C_{1-12})$alkyl, or aryl$(C_{1-6})$alkyl;
Z and $Z^1$ are each 0 or 1;
n is an integar from 2 to 12;
and the polymethylene chain $(CH_2)_n$ may optionally be interrupted by oxygen or sulphur or an aromatic (for instance phenyl or naphthyl) nucleus; or an orally acceptable acid addition salt thereof.

3. A process as claimed in claim 1 or claim 2 in which the cationic antibacterial agent is selected from benzethonium chloride, octenidine, hexetidine, hexamidine, cetyl pyridinium chloride, chlorhexidine or alexidine.

4. A process as claimed in any one of claims 1 to 3 in which the nonionic thickening agent is selected from a $(C_{1-6})$alkylcellulose ether, a hydroxy$(C_{1-6})$alkylcellulose ether, a $(C_{2-6})$alkylene oxide modified $(C_{1-6})$-alkylcellulose ether, or a mixture thereof.

5. A process as claimed in any one of claims 1 to 4 in which the nonionic surfactant is selected from a polyethoxylated sorbitol ester, a polycondensate of ethylene oxide and propylene oxide, a condensate of propylene glycol, a polyethoxylated hydrogenated castor oil or a sorbitan fatty ester; the amphoteric surfactant is selected from a long chain imidazoline derivative, a long chain alkyl betaine or a long chain alkyl amidoalkyl betaine; or cationic surfactant is the D,L-2-pyrrolidone-5-carboxylic acid salt of ethyl-N-cocoyl-L-arginate.

6. A process as claimed in any one of claims 1 to 5 in which the abrasive is an essentially insoluble

compound selected from silica, zinc orthophosphate, plastic particles, alumina, hydrated alumina, and calcium pyrophosphate.

7. A process as claimed in any one of claims 1 to 6 in which the essentially insoluble compound used as the abrasive compound contains less than 1% of anionic impurities, based on the weight of the abrasive.

8. A process as claimed in claim 7 in which the abrasive contains less than 0.5% of anionic impurities, based on the weight of the abrasive.

9. A process as claimed in any one of claims 1 to 8 in which the abrasive is a natural amorphous silica or a synthetic amorphous silica, or a mixture thereof.

10. A process as claimed in claim 9 in which the silica comprises less the 0.5%, by weight of silica, of sulphate anions.

11. A process as claimed in any one of claims 1 to 5 in which the abrasive is a sparingly soluble salt selected from calcium carbonate, calcium phosphates, magnesium carbonate, insoluble sodium metaphosphate, or a mixture thereof.

12. A process as claimed in any one of claims 1 to 5 and 11 in which the agent to suppress anion formation comprises a water soluble salt containing a cation which is the same as the cation of the abrasive and which forms an essentially insoluble or sparingly soluble salt with the anion of the abrasive.

13. A process as claimed in any one of claims 1 to 12 in which the dentifrice further comprises an ionic fluorine-containing compound.

14. A process as claimed in any one of the preceding claims in which the dentifrice further comprises a flavouring agent having an aniseed flavour.

15. A process for preparing a dentifrice comprising a bacteriostatically effective amount.of cetyl pyridinium chloride, a nonionic thickening agent, a nonionic surfactant and an abrasive which is a synthetic amorphous silica comprising less than 1% of anionic impurities, and excluding a dentifrice comprising a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$; which process comprises mixing the ingredients thereof in the required proportions and in any order which is convenient and, thereafter and if necessary, adjusting the pH.

16. The use of a bacteriostatically effective amount of a cationic antibacterial agent; a nonionic thickening agent; a nonionic, cationic or amphoteric surfactant, or a mixture thereof; and an abrasive which comprises either at least one sparingly soluble salt, in combination with an agent to suppress anion formation, or at least one essentially insoluble compound, or a mixture thereof;
and excluding a dentifrice in which the surfactant is essentially a nonionic surfactant and the cationic antibacterial agent is a bis-biguanide and a dentifrice in which the surfactant is essentially a nonionic surfactant and the abrasive consists essentially of a silica which is an amorphous silica which has been treated with hydrofluoric acid, a silica which has been coated with a cationic polymer or a precipitated silica characterised in having from 10-300 parts per million of alkaline earth metal ions, a sulphate ion level below 0.25% and a BET surface area of from 10 to 300 $m^2g^{-1}$, calcium pyrophosphate, trimagnesium phosphate, alumina, hydrated alumina, an aluminium silicate or a mixture thereof; for the manufacture of a dentifrice for oral hygiene. alumina, hydrated alumina, an aluminium silicate or a mixture thereof; for the manufacture of a dentifrice for oral hygiene.